# EUROPEAN PATENT APPLICATION

(11) **EP 3 281 568 A1**
(43) Date of publication of application: **14.02.2018**
(21) Application number: 16776169.1
(22) Date of filing: 11.03.2016
(51) Int. Cl.: A47G 27/00, A61L 2/10

(54) **ANTIBACTERIAL MAT**

(30) Priority: 10.04.2015 ES 201530474
(71) Applicant: Prieto Andreu, Joel, 03330 Crevillente (Alicante) (ES); Prieto Andreu, Jesica, 03330 Crevillente (Alicante) (ES)
(72) Inventor: Prieto Andreu, Joel, 03330 Crevillente (Alicante) (ES); Prieto Andreu, Jesica, 03330 Crevillente (Alicante) (ES)
(74) Representative: Isern-Jara, Nuria
(86) International application number: PCT/ES2016/070155
(87) International publication number: WO 2016/162581

(57) **Abstract**

Disclosed is an antibacterial mat, of the type conventionally disposed at the entrance of an enclosed space, suitable to be trodden on by persons entering said enclosed space, which comprises: a planar cover that can be penetrated by ultraviolet (UV) radiation; and means for generating UV radiation, the means for generating UV radiation being concealed by the cover.

## Description

### OBJECT OF THE INVENTION

The object of the present patent application is to register an antibacterial mat which incorporates notable innovations and advantages over the techniques used hitherto.

More specifically, the invention proposes the development of an antibacterial mat which, because of its particular arrangement, allows for the use of ultraviolet radiation technology to reduce the amount of bacteria being transported on the shoes of persons entering any type of enclosed space or residence.

### BACKGROUND OF THE INVENTION

The possibilities of using ultraviolet radiation as a way to prevent the proliferation of undesirable microorganisms and bacteria, and the resulting application thereof as a germicide and disinfectant for drinks, food, potable water, ventilation, etc. are known in the current state of the art.

Said technology has several advantages, such as the absence of waste or by-products, with no risk whatsoever to people's health.

Also known are the conventional mats at the entrance of houses, residences or enclosed spaces, where people who are about to enter stand and wipe their shoes before doing so.

In other cases, it is common for people to have a closet where they leave their shoes after taking them off.

In any case, it is important to apply a proper germicide and disinfectant to the shoes, given that due to normal use it is possible that such action will be required, in order to prevent the shoes from transporting bacteria.

The present invention contributes to solving the present problem, since it enables an appropriate germicide and disinfectant to be applied to shoes, using the technology of ultraviolet radiation with all of the functions and advantages thereof, all in a simple and comfortable way for the user.

### DESCRIPTION OF THE INVENTION

The present invention has been developed for the purpose of providing an antibacterial mat, of the type conventionally disposed at the entrance of an enclosed space, suitable to be trodden on by persons entering said enclosed space, characterized in that it comprises: a planar cover that can be penetrated by ultraviolet (UV) radiation; and means for generating UV radiation, the means for generating UV radiation being concealed by the cover.

Preferably, in the antibacterial mat, the means for generating UV radiation emit UVC radiation.

Preferably, in the antibacterial mat, the means for generating UV radiation comprise LEDs (Light Emitting Diodes).

Alternatively, in the antibacterial mat, the cover is made of quartz crystal.

Alternatively, in the antibacterial mat, the means for generating UV radiation incorporate an electric battery.

Alternatively, in the antibacterial mat, the means for generating UV radiation are connected to an exterior electrical grid.

Preferably, in the antibacterial mat, the edges of the planar cover have a beveled rubber strip around them.

Preferably, in the antibacterial mat, the cover has indications on the correct positioning and placement for the user's feet.

Preferably, in the antibacterial mat, the cover has two screen-printed shoeprints.

Alternatively, in the antibacterial mat, the means for generating UV radiation incorporate a timer.

Alternatively, in the antibacterial mat, the means for generating UV radiation incorporate a pilot light indicating the time the LEDs are on.

Alternatively, in the antibacterial mat, the means for generating UV radiation incorporate a motion sensor.

Alternatively, in the antibacterial mat, the means for generating UV radiation incorporate photoelectric sensors able to read the size of the surface supported by the cover.

Preferably, the antibacterial mat is embedded or inserted in the floor.

Alternatively, the antibacterial mat is embedded or inserted in a piece of furniture.

Thanks to the present invention, it is possible to apply an appropriate germicide and disinfectant to shoes, using the technology of ultraviolet radiation with all of the functions and advantages thereof, all in a simple and comfortable way for the user.

Other characteristics and advantages of the antibacterial mat will become clear in light of the description of a preferred, though non-exclusive, embodiment, which, by way of a non-limiting example, is illustrated in the accompanying drawings, wherein:

### BRIEF DESCRIPTION OF THE DRAWINGS

Figure 1 is a schematic perspective view of a preferred embodiment of the antibacterial mat of the present invention.
Figure 2 is a schematic perspective view of another preferred embodiment of the antibacterial mat of the present invention.

### DESCRIPTION OF A PREFERRED EMBODIMENT

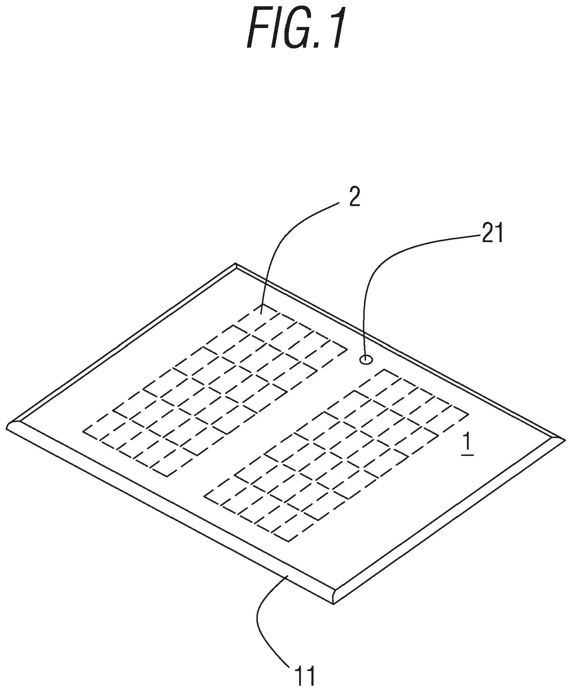

As schematically shown in figure 1, the antibacterial mat of the present invention is of the type that is conventionally disposed at the entrance of an enclosed space and suitable to be trodden on by persons entering said enclosed space.

The antibacterial mat of the invention comprises a planar cover (1) that is transparent to UV radiation; and means for generating UV radiation, the means for generating UV radiation being concealed by the cover (1).

In this preferred embodiment, the cover is made of quartz crystal, and the means for generating UV radiation emit UVC radiation.

The edges of the planar cover (1) have a beveled rubber strip (11) around them to prevent the user from tripping. The cover (1) has suitable proportions in accordance with the enclosed space where it is to be used.

The means for generating UV radiation comprise two LEDs (2) represented by dashes in the figures, given that they are concealed by the cover (1), and uniformly distributed along the surface concealed by the cover (1). For example, in this preferred embodiment, the means for generating UV radiation comprise 30 LED (2) units disposed for each of the user's shoes, which emit UVC radiation at a wavelength of 254 nm with a radiation intensity of 1.5 mW each, meaning a total of 45 mW for each shoe.

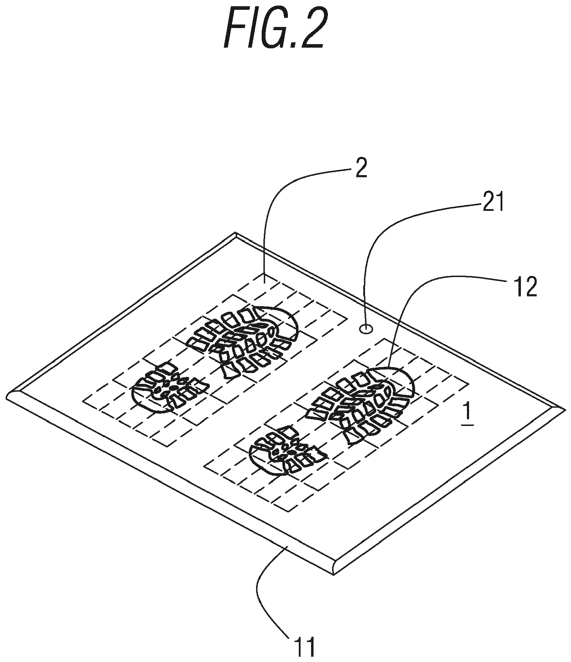

The antibacterial mat of the invention has indications on the cover (1) for the correct positioning and placement of the user's feet. In this preferred embodiment, the cover (1) has screen prints (12) of two shoeprints, indicating the correct positioning of the user's feet, positioned just above the LEDs (2), as schematically shown in figure 2.

In this preferred embodiment, the means for generating UV radiation incorporate an electric battery, such as a 12 V rechargeable lithium battery.

In other preferred embodiments, the means for generating ultraviolet radiation may be connected to an exterior electrical grid.

The means for generating UV radiation may incorporate, in addition to the two LEDs (2), a timer (21) in order to regulate the time the UVC radiation is projected and a light for indicating the time the LEDs (2) are on, as well as a motion sensor for automatically turning on the LEDs (2), and photoelectric sensors, which take a reading of the size of the surface supported by the cover (1), making it so the LEDs (2) will turn on in coordination with the size of the surface of the support shoe. All of these accessories mentioned are known in the state of the art, which is why they are not detailed in the present description.

The antibacterial mat of the invention allows for a reduction to the fullest extent possible of the bacteria that may be brought into any type of enclosed space, whether it be single-family homes, hospitals, hotels, or any other area where such invention may be used. It may also be used for shoe placement when the shoes are not being used by the user, such as inside a closet.

Unlike mats known in the state of the art, the intention with the antibacterial mat of the invention is that they do not become a nest or breeding ground for bacteria, but rather prevent bacteria from being able to reproduce or hibernate.

The antibacterial mat of the invention is useful both for persons who, due to tradition, take off their shoes at the entrance, and for persons who do not take off their shoes before entering.

In the first case, these persons usually have a closet where they leave their shoes, which they use outdoors, and this closet would always be free from bacteria. In the second case, these users could enter without a problem, since their shoes would not be transporting bacteria.

The antibacterial mat is very comfortable for the user, since the time it takes for the bacteria to be exterminated is 5 seconds, which essentially means stepping onto the mat and entering.

The details, shapes, dimensions and other accessory elements, as well as the materials used to manufacture the antibacterial mat of the present invention, may be suitably substituted for others which are technically equivalent, and do not diverge from the essential nature of the invention, nor the scope defined by the claims included below.

## Claims

1. An antibacterial mat, of the type conventionally disposed at the entrance of an enclosed space, suitable to be trodden on by persons entering said enclosed space, **characterized in that** it comprises a planar cover (1) that can be penetrated by ultraviolet (UV) radiation and means for generating UV radiation, the means for generating UV radiation being concealed by the cover (1), said means for generating UV radiation comprising LEDs (2), and said means for generating UV radiation incorporating a motion sensor.

2. The antibacterial mat according to claim 1, **characterized in that** the means for generating UV radiation emit UVC radiation.

3. The antibacterial mat according to claim 1, **characterized in that** the cover (1) is made of quartz crystal.

4. The antibacterial mat according to claim 1, **characterized in that** the means for generating UV radiation incorporate an electric battery.

5. The antibacterial mat according to claim 1, **characterized in that** the means for generating UV radiation are connected to an exterior electrical grid.

6. The antibacterial mat according to claim 1, **characterized in that** the edges of the planar cover (1) have a beveled rubber strip (11) around them.

7. The antibacterial mat according to claim 1, **characterized in that** the cover (1) has indications for the correct positioning and placement of the user's feet.

8. The antibacterial mat according to claim 7, **characterized in that** the cover (1) has screen prints (12) of two shoeprints.

9. The antibacterial mat according to claim 1, **characterized in that** the means for generating UV radiation incorporate a timer (21).

10. The antibacterial mat according to claim 3, **characterized in that** the means for generating UV radiation incorporate a pilot light indicating the time the LEDs (2) are on.

11. The antibacterial mat according to claim 1, **characterized in that** the means for generating UV radiation incorporate photoelectric sensors able to read the size of the surface supported by the cover (1).

12. The antibacterial mat according to claim 1, **characterized in that** it is embedded or inserted in the floor.

13. The antibacterial mat according to claim 1, **characterized in that** it is embedded or inserted in a piece of furniture.
